# EUROPEAN PATENT SPECIFICATION

(11) **EP 2 247 316 B1**
(45) Date of publication and mention of the grant of the patent: **06.11.2013**
(21) Application number: 09715070.0
(22) Date of filing: 26.02.2009
(51) Int. Cl.: A61K 51/04

(54) **IMAGING THE CENTRAL NERVOUS SYSTEM**
BILDGEBUNG DES ZENTRALEN NERVENSYSTEMS
IMAGERIE DU SYSTÈME NERVEUX CENTRAL

(30) Priority: 29.02.2008 GB 0803729; 29.02.2008 US 32442 P
(43) Date of publication of application: 10.11.2010
(73) Proprietor: GE Healthcare Limited, Little Chalfont Buckinghamshire HP7 9NA (GB)
(72) Inventor: JONES, Paul, Alexander, Amersham Buckinghamshire HP7 9LL (GB); WILSON, Ian, Amersham Buckinghamshire HP7 9LL (GB); MORISSON-IVESON, Veronique, Amersham Buckinghamshire HP7 9LL (GB); JONES, Clare, Amersham Buckinghamshire HP7 9LL (GB); WOODCRAFT, John, Amersham Buckinghamshire HP7 9LL (GB); JACKSON, Alex, Amersham Buckinghamshire HP79LL (GB); WYNN, Duncan, Amersham Buckinghamshire HP7 9LL (GB)
(74) Representative: Bannan, Sally
(86) International application number: PCT/EP2009/052280
(87) International publication number: WO 2009/106564

(56) References cited:
- WO-A1-2007/141267
- WO-A1-2008/064432
- STOKES L ET AL: "Characterization of a selective and potent antagonist of human P2X 7 receptors, AZ11645373" BRITISH JOURNAL OF PHARMACOLOGY 20061209 GB, vol. 149, no. 7, 9 December 2006 (2006-12-09), pages 880-887, XP002563405 ISSN: 1476-5381
- MICHEL A D ET AL: "Negative and positive allosteric modulators of the P2X7 receptor" BRITISH JOURNAL OF PHARMACOLOGY 200802 GB, vol. 153, no. 4, February 2008 (2008-02), pages 737-750, XP002563406 ISSN: 1476-5381
- NELSON DEREK W ET AL: "Structure-Activity Relationship Studies on a Series of Novel, Substituted 1-Benzyl-5-phenyltetrazole P2X7 Antagonists" JOURNAL OF MEDICINAL CHEMISTRY, AMERICAN CHEMICAL SOCIETY, WASHINGTON, US, vol. 49, no. 12, 1 January 2006 (2006-01-01), pages 3659-3666, XP008098135 ISSN: 0022-2623 [retrieved on 2006-05-23] cited in the application
- BARALDI P G ET AL: "Agonists and antagonists acting at P2X7 receptor" CURRENT TOPICS IN MEDICINAL CHEMISTRY 2004 NL, vol. 4, no. 16, 2004, pages 1707-1717, XP8117103 ISSN: 1568-0266 cited in the application

## Description

### Technical Field of the Invention

The present invention relates to the field of purinergic P2 receptors. More particularly, the present invention relates to novel purinergic P2X₇ receptor *in vivo* imaging agents, their production and intermediates thereof. In further detail, the present invention relates to the use of the *in vivo* imaging agents of the invention in methods to provide information useful in the diagnosis of disease states in which P2X₇ receptor expression is implicated.

### Description of Related Art

The P2X₇ receptor is a cation-selective ion channel directly gated by extracellular ATP (the only known physiological ligand) and a few pharmacological ATP analogues (North 2002 Physiol. Rev. 82:1013-1067), The release of ATP from damaged cells and the subsequent activation of purinergic P2X₇ receptors located on hematopoietic cells (such as microglia, macrophages and lymphocytes) is crucial to the inflammatory cascade (Ferrari D et al 2006 J. Immunol. 176:3877-83). The cation movement associated with the opening of the plasma membrane P2X₇ channel is necessary for the maturation and release of the main pro-inflammatory cytokine, interleukin-1ß (IL-1ß). While the expression of P2X₇ is low in normal tissue, during inflammation (whether central or peripheral) there is a large increase in P2X₇ reactivity on cells in the surrounding area.

In the central nervous system (CNS), increases in P2X₇ have been characterised following the experimental inducement of stroke (Franke et al 2004 J. Neuropathol. Exp. Neurol. 63:686-99); multiple sclerosis (MS) (Yiangou et al 2006 BMC. Neurol. 6:12); amyotrophic lateral sclerosis (ALS) (Yiangou *et al 2006 supra*); epilepsy (Rappold et al 2006 Brain Res. 1089:171-8); and, in a transgenic, amyloidic Alzheimer's disease mouse (Parvathenani et al 2003 J. Biol. Chem. 278:13309-17). In the periphery, P2X₇ receptor upregulation has been shown to accompany neuropathic pain (Chessell et at 2005 Pain 114:386-96); polycystic kidney disease (Franco-Martinez et al 2006 Clin. Exp. Immunol. 146:253-61); and, tuberculosis (Hillman et al 2005 Nephron. Exp. Nephrol. 101:e24-30).

P2X₇ upregulation has also been shown in a variety of cancers, e.g. cervical, uterine, prostate, breast and skin cancers and leukaemias, both in experimental models and in patients (Feng et al 2006 J. Biol. Chem. 281:17228-37; Greig et al 2003 J. Invest. Dermatol. 121:315-327; Slater et al 2004 Histopathology 44:206-215 Slater et al 2004 Breast Cancer Res. Treat. 83:1-10; Zhang et al 2004 Leuk. Res 28:1313-1322; Li et al 2006 Cancer Epidemiol. Biomarkers Prev. 15:1906-13).

A number of compound classes have been synthesised from different structural backbones to generate therapeutic P2X₇ antagonists. A review of agonists and antagonists acting at the P2X₇ receptor has been published by Baraldi et al (2004 Curr. Topics Med. Chem. 4:1707-17). The compounds disclosed therein are discussed as being potentially useful therapeutic agents.

Small molecule P2X₇ binding compounds have also been disclosed in relation to *in vivo* imaging applications. WO 2007/141267 is primarily related to treatment and provides pyrazole derivatives that are P2X₇ antagonists for the treatment of pain, inflammation and neurodegeneration. Isotopically-labelled versions of the compounds are mentioned as useful for *in vivo* imaging by single-photon emission tomography (SPECT) or PET, although no detail is provided in WO 2007/141267 as to how to obtain such isotopically-labelled versions. WO 2007/109154 and WO 2007/109192 are also primarily related to treatment and disclose bicycloheteroaryl compounds as P2X₇ modulators. Isotopic variants of these comprising ¹¹C, ¹⁸F, ¹⁵O or ¹³N are mentioned as useful in PET studies of substrate receptor occupancy, although there is no description in either WO 2007/109154 or WO 2007/109192 of how to obtain these isotopic variants. WO 2008/064432 has a priority date earlier than, and a publication date later than, that of the present invention. WO 2008/064432 discloses polycyclic compounds for the diagnosis, treatment or monitoring of disorders in which the P2X₇ receptor is implicated. Compounds of WO 2008/064432 that were tested in a P2X₇ receptor functional assay demonstrated that the compounds were antagonists of the P2X₇ receptor. The compounds of WO 2008/064432 may be radiolabelled with an isotope suitable for *in vivo* imaging, e.g. by SPECT or PET, and have physiochemical properties particularly suitable for *in vivo* imaging studies.

There is scope for an alternative *in vivo* imaging agent suitable for imaging the P2X₇ receptor to facilitate the diagnosis of disease states associated with the P2X₇ receptor, in particular those of the central nervous system (CNS).

### Summary of the Invention

The present invention provides novel compounds which may be used as *in vivo* imaging agents. The *in vivo* imaging agents of the invention are particularly useful in a method to image the expression of P2X₇ receptors in the CNS of a subject, as a means to facilitate the diagnosis of a range of disease states.

### Detailed Description of the Invention

### IN VIVO IMAGING AGENT

In one aspect, the present invention provides an *in vivo* imaging agent suitable for *in vivo* imaging of the central nervous system (CNS) of a subject, wherein said *in vivo* imaging agent is a compound of Formula II, or a salt or solvate thereof wherein:
Formula II is defined as follows: 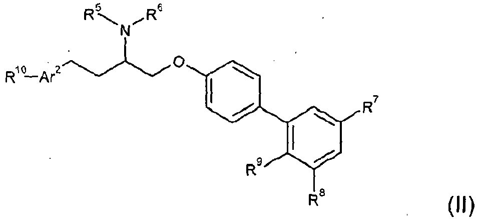
   wherein one of R⁵-R¹⁰ comprises an *in vivo* imaging moiety which is a gamma-emitting radioactive halogen or a positron-emitting radioactive non-metal, and wherein:
   R⁵ and R⁶ are independently selected from hydrogen, C₁₋₆ alkyl, C₁₋₆ fluoroalkyl, C₁₋₆ acyl, C₁₋₆ fluoroacyl, C₁₋₆ carboxylic acid alkyl ester, C₁₋₆ alkoxy, C₁₋₆ fluoroalkoxy; or R¹ and R², taken together with the nitrogen to which they are attached, form a 5- or 6-membered nitrogen-containing heterocycle optionally comprising another heteroatom selected from nitrogen, sulfur or oxygen, and optionally having 1 or 2 oxo groups on the ring;
   R⁷-R⁹ are independently selected from hydrogen, halo, nitro, C₁₋₆ alkyl, C₁₋₆ haloalkyl, C₅₋₆aryl, or C₅₋₆haloaryl;
   R¹⁰ is selected from hydrogen, hydroxyl, nitro, halo, or is the group C(=O)NR¹¹R¹² wherein R¹¹ and R¹² are as defined for R⁵ and R⁶; and,
   Ar² is a 5- to 6-membered aryl group having 0-3 heteroatoms selected from nitrogen, oxygen and sulfur.

The term *"in* *vivo* imaging agent" refers to a compound which can be used to detect a particular physiology or pathophysiology in a living subject.by means of its administration to said subject and subsequent detection within said subject, wherein detection is carried out external to said subject.

In order to be "suitable for *in vivo* imagine of the central nervous system (CNS)" an *in vivo* imaging agent needs to be able to cross the blood-brain barrier (BBB). The "CNS" is that part of the nervous system of a subject comprising the brain and spinal cord that is covered by the meninges. The generally accepted biophysical/physicochemical models of BBB penetration have as their primary determinants for passive transport: the solute's lipophilicity; hydrogen-bond desolvation potential; pKa/charge; and, molecular size. For example, a suitable lipophilicity value for a compound to penetrate the BBB would be logP in the range 1.0-4.5, preferably 2.0-3.5.

The "subject" of the invention is preferably a mammal, most preferably an intact mammalian body *in vivo.* In an especially preferred embodiment, the subject of the invention is a human.

In the term "salt or solvate thereof", a suitable salt may be selected from (i) physiologically acceptable acid addition salts such as those derived from mineral acids, for example hydrochloric, hydrobromic, phosphoric, metaphosphoric, nitric and sulphuric acids, and those derived from organic acids, for example tartaric, trifluoroacetic, citric, malic, lactic, fumaric, benzoic, glycollic, gluconic, succinic, methanesulphonic, and para-toluenesulphonic acids; and (ii) physiologically acceptable base salts such as ammonium salts, alkali metal salts (for example those of sodium and potassium), alkaline earth metal salts (for example those of calcium and magnesium), salts with organic bases such as triethanolamine, N-methyl-D-glucamine, piperidine, pyridine, piperazine, and morpholine, and salts with amino acids such as arginine and lysine. A suitable solvate may be selected from those formed with ethanol, water, saline, physiological buffer and glycol.

The term "comprises an *in vivo* imagin moiety" means that a functional group of said *in vivo* imaging agent of Formula II, as defined herein, comprises an *in vivo* imaging moiety. When a functional group comprises an imaging moiety, this means that the 'imaging moiety' forms part of the chemical structure, and is a radioactive isotope present at a level significantly above the natural abundance level of said isotope. Such elevated or enriched levels of isotope are suitably at least 5 times, preferably at least 10 times, most preferably at least 20 times; and ideally either at least 50 times the natural abundance level of the isotope in question, or present at a level where the level of enrichment of the isotope in question is 90 to 100%. Examples of such functional groups include iodophenyl groups with elevated levels of ¹²³I, CH₃ groups with elevated levels of ¹¹C, and fluoroalkyl groups with elevated levels of ¹⁸F, such that the imaging moiety is the isotopically labelled ¹¹C or ¹⁸F atom within the chemical structure. More detailed discussion of how these and other suitable functional groups are incorporated into the *in vivo* imaging agents of the invention is given later on in this description.

A suitable "*in vivo* imaging moiety" of the present invention is either a gamma-emitting radioactive halogen or a positron-emitting radioactive non-metal. When the *in vivo* imaging moiety is a gamma-emitting radioactive halogen, the radiohalogen is suitably chosen from ¹²³I, ¹³¹I or ⁷⁷Br. ¹²⁵I is specifically excluded as it is not suitable for *in vivo* imaging use. A preferred gamma-emitting radioactive halogen for *in vivo* imaging is ¹²³I. When the imaging moiety is a positron-emitting radioactive non-metal, suitable such positron emitters include: ¹¹C, ¹³N, ¹⁵O, ¹⁷F, ¹⁸F, ⁷⁵Br, ⁷⁶Br or ¹²⁴I. Preferred positron-emitting radioactive non-metals are ¹¹C, ¹³N, ¹⁸F and ¹²⁴I. especially ¹¹C and ¹⁸F, most especially ¹⁸F.

A compound that is a "ligand for the P2X₇ receptor" demonstrates at least 40% (preferably at least 60% and most preferably at least 70%) inhibition of the function of an agonist to form a non-selective pore in HEK.293 cells (see Michel et al, B. J. Pharmacol. 1998; 125: 1194-1201). In terms of binding affinity, a ligand for the P2X₇ receptor has a K_{d} or Kᵢ of between 0.01 and 100nM, preferably between 0.01 and 10nM, and most preferably between 0.01 and 1 nM (as measured by: Humphreys et al 1998 Molecular Pharmacology, 54:22-32; Chessell et al 1998 British Journal of Pharmacology, 124: 1314-1320). In conjunction with binding affinity for the P2X₇ receptor, the in *vivo* imaging agents of the invention preferably have no affinity up to 10µM for other P2 receptors. The in *vivo* imaging agent of the invention is preferably an antagonist for the P2X₇ receptor.

Unless otherwise specified, the term "alkyl" alone or in combination, means a straight-chain or branched-chain alkyl radical containing from 1 to 6 carbon atoms, preferably 1 to 3 carbon atoms. Examples of such radicals include methyl, ethyl, n-propyl, isopropyl, n-butyl, isobutyl, sec-butyl, tert-butyl, pentyl, iso-amyl, hexyl.

Unless otherwise specified, the term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert- butoxy.

"Aryl" means aromatic rings or ring systems having 5 to 12 carbon atoms, preferably 5 to 6 carbon atoms, in the ring system, e.g. phenyl or naphthyl. A "heteroaryl" substituent is an aryl as defined herein wherein at least one of the carbon atoms of the ring has been replaced with a heteroatom selected from N, S or O.

"Acyl" is defined as any group comprising the radical RC(=O), wherein R is an alkyl group as defined above. "Acetyl" is an acyl group wherein the R group is methyl.

The term "halo" means a substituent selected from fluorine, chlorine, bromine or iodine. "Haloalkyl", "haloacyl", "hafo alkoxy" and "haloaryl" are alkyl, acyl, alkoxy and aryl groups, respectively, as defined above substituted with one or more halo groups.

"Hydroxyl" is the group -OH. The term "hydroxyalkyl" represents an alkyl group as defined above substituted with one or more hydroxyl groups.

"Nitro" is the group -NO₂.

"Oxo" is the group =O.

The term "alkylene" means a bivalent linker moiety of the formula (CH₂)ₙ wherein, unless otherwise specified, n is preferably between 1 and 6.

The term "heterocycle" means an aliphatic or aromatic C₅₋₁₂ cyclic radical wherein at least one nitrogen, oxygen or sulfur ring member. C₅₋₁₀ cyclic radicals are preferred.

The term "carboxylic acid alkyl ester" is a group defined by the formula R'C(=O)OR" wherein R' and R" are C₁₋₆ alkyl groups as defined above.

In a preferred embodiment, the *in vivo* imaging agent of the invention is a compound of Formula II*: 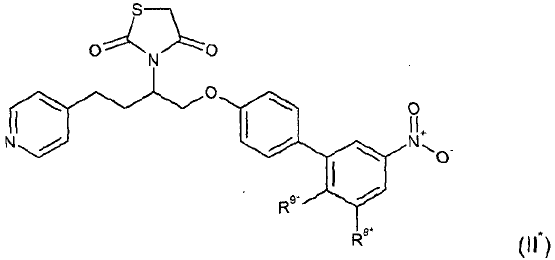 wherein one of R^{8*} and R^{9*} is ¹⁸F and the other is hydrogen.

### METHOD OF SYNTHESIS & PRECURSOR

The *in vivo* imaging agents of the invention may be obtained by reaction of a suitable source of the desired *in vivo* imaging moiety with a precursor compound.

A "precursor compound" comprises an unlabelled derivative of a compound of Formula II as defined above, designed so that chemical reaction with a convenient chemical form of the imaging moiety occurs site-specifically; can be conducted in the minimum number of steps (ideally a single step); and without the need for significant purification (ideally no further purification), to give the desired *in vivo* imaging agent of Formula II as defined herein. Such precursor compounds are synthetic and can conveniently be obtained in good chemical purity. The precursor compound may optionally comprise a protecting group for certain functional groups of the precursor compound.

By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection, the desired *in vivo* imaging agent of Formula II as defined herein is obtained. Protecting groups are well known to those skilled in the art and are suitably chosen from, for amine groups: BOC (where BOC is tert-butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl); and for carboxyl groups: methyl ester, *tert*-butyl ester or benzyl ester. For hydroxyl groups, suitable protecting groups are: methyl, ethyl or *tert*-butyl; alkoxymethyl or alkoxyethyl; benzyl; acetyl; benzoyl; trityl (Trt) or trialkylsilyl such as tetrabutyldimethylsilyl. For thiol groups, suitable protecting groups are: trityl and 4-methoxybenzyl. The use of further protecting groups are described in Protective Groups in Organic Synthesis', Theorodora W. Greene and Peter G. M. Wuts, (Third Edition, John Wiley & Sons, 1999).

Where the imaging moiety is radioiodine, the *in vivo* imaging agent of Formula II as defined herein can be obtained by means of a precursor compound comprising a derivative which either undergoes electrophilic or nucleophilic iodination or undergoes condensation with a labelled aldehyde or ketone. Examples of the first category are:
(a) organometallic derivatives such as a trialkylstannane (e.g. trimethylstannyl or tributylstannyl), or a trialkylsilane (e.g. trimethylsilyl) or an organoboron compound (e.g. boronate esters or organotrifluoroborates);
(b) a non-radioactive alkyl bromide for halogen exchange or alkyl tosylate, mesylate or triflate for nucleophilic iodination;
(c) aromatic rings activated towards nucleophilic iodination (e.g. aryl iodonium salt aryl diazonium, aryl trialkylammonium salts or nitroaryl derivatives).

Preferred such precursor compounds comprise: a non-radioactive halogen atom such as an aryl iodide or bromide (to permit radioiodine exchange); an organometallic precursor compound (e.g. trialkyltin, trialkylsilyl or organoboron compound); or an organic precursor such as triazenes or a good leaving group for nucleophilic substitution such as an iodonium salt. Preferably for radioiodination, the precursor compound comprises an organometallic precursor compound, most preferably trialkyltin.

Precursor compounds and methods of introducing radioiodine into organic molecules are described by Bolton [J.Lab.Comp.Radiopharm., 45, 485-528 (2002)]. Suitable boronate ester organoboron compounds and their preparation are described by Kabalka et al [Nucl.Med.Biol., 29, 841-843 (2002) and 30, 369-373(2003)]. Suitable organotrifluoroborates and their preparation are described by Kabalka et al [Nucl.Med.Biol., 31, 935-938 (2004)].

Examples of aryl groups to which radioactive iodine can be attached are given below:

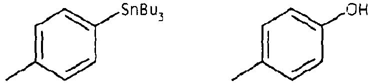

Both contain substituents which permit facile radioiodine substitution onto the aromatic ring.

Alternative substituents containing radioactive iodine can be synthesised by direct iodination via radiohalogen exchange, e.g.

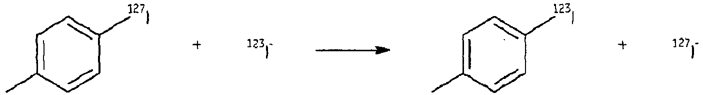

The radioiodine atom is preferably attached via a direct covalent bond to an aromatic ring such as a benzene ring, or a vinyl group since it is known that iodine atoms bound to saturated aliphatic systems are prone to *in vivo* metabolism and hence loss of the radioiodine.

One approach to labelling with ¹¹C is to react a precursor compound which is the desmethylated version of a methylated compound with [¹¹C]methyl iodide. It is also possible to incorporate ¹¹C by reacting a Grignard reagent of the particular hydrocarbon of the desired *in vivo* imaging agent with [¹¹C]CO₂ to obtain a ¹¹C reagent that reacts with an amine group in the precursor compound to result in the ¹¹C-labelled *in vivo* imaging agent of interest.

¹¹C could also be introduced as a methyl group on an aromatic ring, in which case the precursor compound would include a trialkyltin group or a B(OH)₂ group.

As the half-life of ¹¹C is only 20.4 minutes, it is important that the intermediate ¹¹C moieties have high specific activity and, consequently, are produced using a reaction process which is as rapid as possible.

A thorough review of such ¹¹C-labelling techniques may be found in Antoni et al "Aspects on the Synthesis of 11C-Labelled Compounds" in Handbook of Radiopharmaceuticals, Ed. M.J. Welch and C.S. Redvanly (2003, John Wiley and Sons).

Radiofluorination may be carried out via direct labelling using the reaction of ¹⁸F-fluoride with a suitable chemical group in a precursor compound having a good leaving group, such as an alkyl bromide, alkyl mesylate or alkyl tosylate. ¹⁸F can also be introduced by alkylation of N-haloacetyl groups with a ¹⁸F(CH₂)₃OH reactant, to give -NH(CO)CH₂O(CH₂)₃ ¹⁸F derivatives. For aryl systems, ¹⁸F-fluoride nucleophilic displacement from an aryl diazonium salt, aryl nitro compound or an aryl quaternary ammonium salt are suitable routes to aryl-¹⁸F derivatives.

A ¹⁸F-labelled *in vivo* imaging agent of the invention may be obtained by formation of ¹⁸F fluorodialkylamines and subsequent amide formation when the ¹⁸F fluorodialkylamine is reacted with a precursor containing, e.g. chlorine, P(O)Ph₃ or an activated ester.

Further details of synthetic routes to ¹⁸F-labelled derivatives are described by Bolton, J.Lab.Comp.Radiopharm., 45, 485-528 (2002).

### Biphenyls

Precursors for the synthesis of *in vivo* imaging agents of Formula II may be obtained using methods analogous to those presented by Alcaraz et al (2003 Bioorg. Med. Chem. Lett. 13: 4043-6). A precursor compound suitable for the synthesis of *in vivo* imaging agents of Formula II is a compound of Formula IIa: 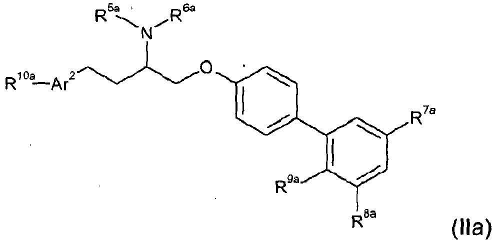 wherein:
wherein:
   R^{5a} and R^{6a} are as defined above for R⁵ and R⁶ of Formula II, respectively; and,
   one of R^{7a}-R^{10a} represents a precursor group, and the remainder are as defined above for R⁷-R¹⁰ of Formula II, respectively.

A "precursor group" is one which reacts with a convenient chemical form of the imaging moiety to incorporate the imaging moiety site-specifically. Suitable such precursor groups have already been discussed in the description above. For example, such precursor groups include iodo, hydroxyl, nitro, iodonium salt, bromo, mesylate, tosylate, trialkyltin, B(OH)₂, and trialkylammonium salt.

Preferably, said precursor compound of Formula IIa is a compound of Formula IIa*: 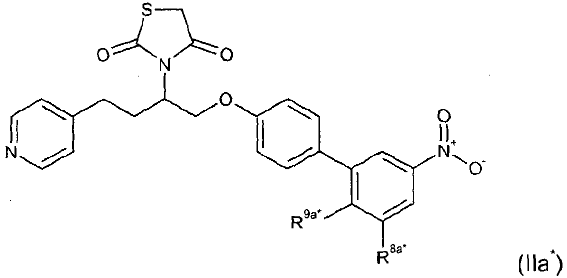 wherein one of R^{8a*} and R^{9a*} is a precursor group, and the other is hydrogen.

**Table I below provides examples of some particular precursor compounds and their respective in vivo imaging agents:**

| Precursor Compound | Imaging Agent | % Inhibition | |
|---|---|---|---|
| | | *10µM* | *100nM* |
| 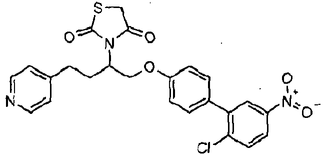 | 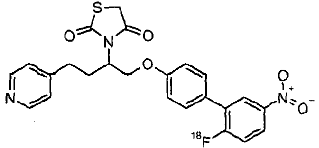 | 72.0 | 22.0 |
| Precursor Compound 2 | Imaging Agent 2 | | |
| 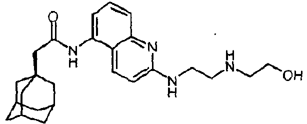 | 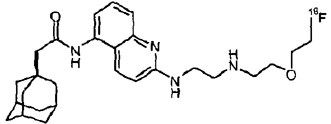 | 62.0 | 0.00 |
| Precursor Compound 3 | Imaging Agent 3 | | |
| 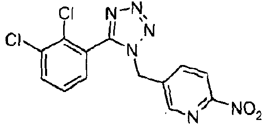 | 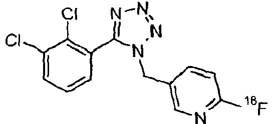 | 40.5 | 17.4 |
| Precursor Compound 4 | Imaging Agent 4 | | |
| 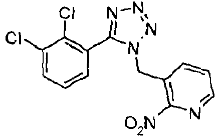 | 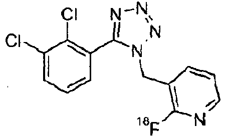 | 69.6 | 10.0 |
| Precursor Compound 5 | Imaging Agent 5 | | |

Non-radioactive versions of the Imaging Agents illustrated in Table I were screened in a P2X₇ receptor functional assay. This assay is described in Example 9 and is based upon the ability of the P2X₇ receptor to form a non-selective pore in P2X₇ transfected HEK.293 cells upon activation with an agonist, thereby allowing dye to permeate the cells. The non selective P2X channel antagonist used as a reference inhibitor for the evaluation of the non-radioactive compound of the invention was pyridoxal-phosphate-6-azophenyl-2',4'-disulfonate (PPADS), and the results of the assay are provided in Table I above. The non-radioactive versions of the imaging agents were found to inhibit P2X₇ function at 10 µM and generally at 100 nM concentrations to a similar degree compared to PPADS (the reference compound).

The synthetic routes used to obtain the Imaging Agents illustrated in Table I, along with their non-radioactive equivalents, are provided in Examples 1-8.

The precursor compound may be conveniently provided as part of a kit, for example for use in a radiopharmacy. Such a kit comprises the precursor compound as defined herein in a sealed container. The sealed container preferably permits maintenance of sterile integrity and/or radioactive safety, plus optionally an inert headspace gas (e.g. nitrogen or argon), whilst permitting addition and withdrawal of solutions by syringe. A preferred sealed container is a septum-sealed vial, wherein the gas-tight closure is crimped on with an overseal (typically of aluminium). Such sealed containers have the additional advantage that the closure can withstand vacuum if desired e.g. to change the headspace gas or degas solutions.

The precursor compound for use in the kit may be employed under aseptic manufacture conditions to give the desired sterile, non-pyrogenic material. The precursor compound may alternatively be employed under non-sterile conditions, followed by terminal sterilisation using e.g. gamma-irradiation, autoclaving, dry heat or chemical treatment (e.g. with ethylene oxide). Preferably, the precursor compound is provided in sterile, non-pyrogenic form. Most preferably the sterile, non-pyrogenic precursor compound is provided in the sealed container as described above.

Preferably, all components of the kit are disposable to minimise the possibilities of contamination between runs and to ensure sterility and quality assurance.

### AUTOMATED SYNTHESIS AND CASSETTE

In a preferred aspect, the method of synthesis of the present invention is automated. [¹⁸F]-radiotracers in particular are now often conveniently prepared on an automated radiosynthesis apparatus. There are several commercially-available examples of such apparatus, including Tracerlab^{™} and Fastlab^{™} (both available from GE Heathcare). The radiochemistry is performed on the automated synthesis apparatus by fitting the cassette to the apparatus. The cassette normally includes fluid pathways, a reaction vessel, and ports for receiving reagent vials as well as any solid-phase extraction cartridges used in post-radiosynthetic clean up steps.

In a yet further aspect, the present invention provides a cassette which can be plugged into a suitably adapted automated synthesiser for the automated synthesis of the *in vivo* imaging agent of the invention.

The cassette for the automated synthesis of the *in vivo* imaging agent of the invention comprises:
(i) a vessel containing a precursor compound as defined herein; and
(ii) means for eluting the vessel with a suitable source of an *in vivo* imaging moiety, said *in vivo* imaging moiety as defined herein.

The cassette may additionally comprise:
(iii)an ion-exchange cartridge for removal of excess *in vivo* imaging moiety; and optionally,
(iv)a cartridge for deprotection of the resultant radiolabelled product to form an *in vivo* imaging agent as defined herein.

The reagents, solvents and other consumables required for the synthesis may also be included together with a data medium, such as a compact disc carrying software, which allows the automated synthesiser to be operated in a way to meet the end user's requirements for concentration, volumes, time of delivery etc.

### METHOD OF IMAGING

The *in vivo* imaging agents of the invention are particularly useful for the assessment by *in vivo* imaging of the number and/or location of P2X₇ receptors in the CNS of a subject. In a further aspect therefore, the present invention provides a method of imaging a subject to facilitate the determination of the presence, location and/or amount of P2X₇ receptors in the CNS of a subject, said method comprising the following steps:
(i) providing a subject to whom a detectable quantity of an *in vivo* imaging agent as defined herein has been administered;
(ii) allowing the *in vivo* imaging agent to bind to P2X₇ receptors in said subject;
(iii) detection of signals emitted by said *in vivo* imaging agent by an *in vivo* imaging method; and,
(iv) generation of an image representative of the location and/or amount of said signals.

The method of the invention begins by "providing" a subject to whom a detectable quantity of an *in vivo* imaging agent of the invention has been administered. Since the ultimate purpose of the method is the provision of a diagnostically-useful image, administration to the subject of the *in vivo* imaging agent of the invention can be understood to be a preliminary step necessary for facilitating generation of said image.

The properties of the *in vivo* imaging agent of the invention make it suitable for crossing the BBB and binding to P2X₇ receptors within the CNS. Therefore, in the method of the invention the detection and generation steps are carried out on the CNS of said subject, preferably the brain.

The method of the invention may be used to study the location and/or amount of P2X₇ receptor in a healthy subject. However, the method is particularly useful when said subject is known or suspected to have a pathological condition associated with abnormal expression of P2X₇ receptors, and specifically where said abnormal expression is in the CNS (a "P2X₇ condition"). Such conditions include stroke, multiple sclerosis, amyotrophic lateral sclerosis, epilepsy, and Alzheimer's disease, and the pathophysiology of each comprises neuroinflammation. The term "neuroinflammation" refers to the fundamentally inflammation-like character of microglial and astrocytic responses and actions in the CNS. These responses are central to the pathogenesis and progression of a wide variety of neurological disorders including stroke, epilepsy, Parkinson's disease, multiple sclerosis (MS), amyotrophic lateral sclerosis (ALS), Alzheimer's disease and Huntington's disease. Consequently, the image generated by the method of the invention finds use in providing guidance to a clinician in the diagnosis of such disorders.

In an alternative aspect, the present invention provides an in vivo imaging agent for use in a method of diagnosis, comprising steps (i)-(iv) of the method of imaging as defined above, and further comprising the following step:
(v) evaluating the image generated in step (iv) to diagnose a pathological condition associated with abnormal expression of P2X₇ receptors in the CNS (a "P2X₇ condition").

The P2X₇ condition of step (v) is any one of those described herein. The evaluating step is carried out by a doctor or a vet, i.e. a person suitably qualified to make a clinical diagnosis. Such a diagnosis represents a deductive medical or veterinary decision, which is made for the purpose of making a decision about whether any treatment is required to restore the subject to health.

In a further alternative embodiment, the method may include the preliminary step of administering the *in vivo* imaging agent of the invention to the subject. Administration of the *in vivo* imaging agent of the invention is preferably carried out parenterally, and most preferably intravenously. The intravenous route represents the fastest way of delivering the *in vivo* imaging agent of the invention across the BBB and into contact with P2X₇ receptors in the CNS. Preferred embodiments of said *in vivo* imaging agent and subject are as previously defined.

The *in vivo* imaging agent of the invention is preferably administered as a "radiopharmaceutical composition" which comprises the *in vivo* imaging agent of Formula II together with a biocompatible carrier, in a form suitable for mammalian administration.

The "biocompatible carrier" is a fluid, especially a liquid, in which the *in vivo* imaging agent of Formula II is suspended or dissolved, such that the radiopharmaceutical composition is physiologically tolerable, i.e. can be administered to the mammalian body without toxicity or undue discomfort. The biocompatible carrier medium is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols). The biocompatible carrier medium may also comprise biocompatible organic solvents such as ethanol. Such organic solvents are useful to solubilise more lipophilic compounds or formulations. Preferably the biocompatible carrier medium is pyrogen-free water for injection, isotonic saline or an aqueous ethanol solution. The pH of the biocompatible carrier medium for intravenous injection is suitably in the range 4.0 to 10.5.

Such radiopharmaceutical compositions are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose", and are therefore preferably a disposable or other syringe suitable for clinical use. The pre-filled syringe may optionally be provided with a syringe shield to protect the operator from radioactive dose. Suitable such radiopharmaceutical syringe shields are known in the art and preferably comprise either lead or tungsten.

The radiopharmaceutical composition may be prepared from a kit. Alternatively, they may be prepared under aseptic manufacture conditions to give the desired sterile product. The radiopharmaceutical composition may also be prepared under non-sterile conditions, followed by terminal sterilisation using e.g. gamma-irradiation, autoclaving, dry heat or chemical treatment (e.g. with ethylene oxide).

The method of imaging of the present invention may also be employed as a research tool. For example, for the performance of competition studies which allow the interaction of a drug with P2X₇ receptors to be studied. Such studies include dose-occupancy studies, determination of optimal therapeutic dose, drug candidate selection studies, and determination of P2X₇ receptor distribution in the tissue of interest.

In an alternative embodiment, the method of the invention is carried out repeatedly, e.g. before, during and after treatment with a drug to combat a P2X₇ condition, In this way, the effect of said treatment can be monitored over time.

Also provided by the present invention is an *in vivo* imaging agent of the invention for use in medicine, and in particular for use in a method for the determination of the presence, location and/or amount of inflammation in the CNS of a subject. Suitable and preferred embodiments of said *in vivo* imaging agent, method and subject are as previously defined.

In a further aspect of the invention, the *in vivo* imaging agent of the invention may be employed for use in the preparation of a medicament for the determination of the presence, location and/or amount of inflammation in the CNS of a subject. Suitable and preferred embodiments of said *in vivo* imaging agent and said subject are as previously defined herein.

Detailed methods for the synthesis of particular *in vivo* imaging agents of the invention are provided in the following Examples.

### Brief Description of the Examples

Examples 1, 3, 5 and 7 describe the synthesis of Non-radioactive Imaging Agents 2-5, respectively.

Examples 2, 4, 6 and 8 describe the synthesis of Imaging Agents 2-5, respectively.

Example 9 described the assay used to evaluate binding to the P2X₇ receptor.

### Abbreviations used in the Examples

- AIBN: azobisisobutyronitrile
- ATP: adenosine triphosphate
- BOC: *tert*-butoxycarbonyl
- Bz-ATP: 2' and 3'-O-(4-benzoylbenzoyl)-ATP
- DEAD: diethyl azodicarboxylate
- DMSO: dimethyl sulfoxide
- DNA: deoxyribonucleic acid
- EDCl: 1-ethyl-3-(3-dimethylaminopropyl) carbodiimide
- HEPES: 4-(2-hydroxyethyl)-1-piperazineethanesulfonic acid
- HPLC: high-performance liquid chromatography
- IC50: half maximal inhibitory concentration
- LDA: lithium diisopropylamide
- MeOH: methanol
- NBS: *N*-bromosuccinimide
- PPADs: pyrdoxalphosphate-6-azophenyl-2'4'-disulphonic acid
- RNA: ribonucleic acid
- RT: room temperature
- THF: tetrahydrofuran

### Examples

### Example 1: Synthesis of Non-radioactive Imaging Agent 2

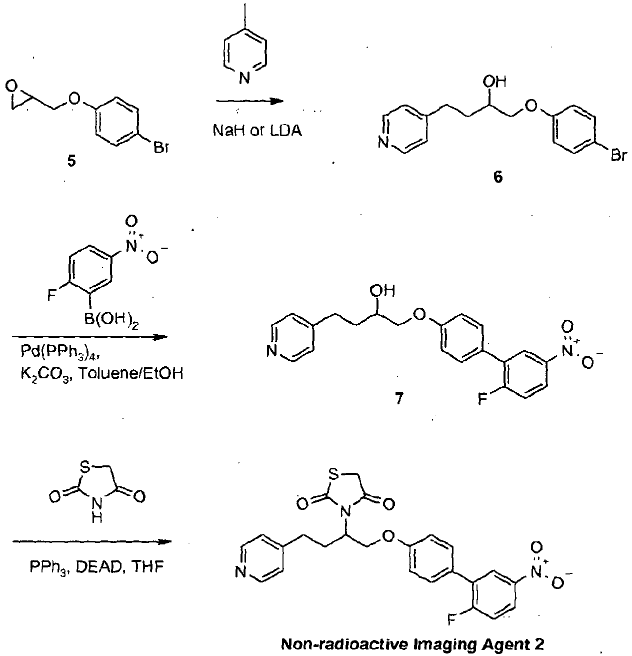

### 3(i) 2-((4-Bromophenoxy)methy/) oxirane (5)

To an oven dried round bottom two-neck flask was added 4-bromophenol (15g 86.7 mmol). Potassium carbonate (14.3 g, 1.2 eqv) was added and the mixture was stirred at room temperature for 10 minutes. Epichlorohydrin (39.8 g, 430 mmol) was added and the mixture heated at 120°C for 3h. The reaction mass was concentrated under reduced pressure to remove the excess of epichlorohydrin. Added water (100 mL) to the reaction mass and extracted with ethyl acetate (4 x 50 mL). The combined organic layers were dried over anhydrous sodium sulphate, filtered and concentrated under reduced pressure. The crude residue was purified using column chromatography on silica gel using hexane and ethyl acetate as eluent to give the desired product (13.6 g, 69% yield).
¹H-NMR: (300MHz, CDCl₃) δ7.40 (d, 2H, J=9Hz), 6.83 (d, 2H, J=9Hz), 4.23 (dd, 1 H, J=12Hz), 3.93 (dd, 1H, J=9Hz), 3.37 (m, 1H), 2.93(t, 1 H), 2.77 (m, 1 H), LCMS: Mass Found [M+H]⁺ 227/229 Calcd for C₉H₉ BrO₂ 228.

### 3(ii) 1-(4-Bromophenoxy)-4-(pyridin-4-yl) butan-2-ol (6)

4-Picoline (1.0 g, 10.74 mmol) was added to an oven dried round bottom flask and flushed with nitrogen. To it added anhydrous THF (8ml). The mixture was kept under nitrogen and cooled to -78°C and stirred at this temperature for 30 minutes. A solution of butyllithium (4.8 mL of 2.5M) was added and the mixture stirred for another 30 minutes. The reaction mass was then added to round bottomed flask containing 2-((4-brornophenoxy)methyl) oxirane (2.4 g 10.48 mmol) at 0°C. The entire reaction mass was quenched by the addition of saturated aqueous ammonium chloride (25 mL) and extracted with dichloromethane (3 x 10 mL). The combined extracts were dried over anhydrous sodium sulphate, filtered and evaporated under reduced pressure. The crude product was purified by column chromatography on silica gel using hexane and ethyl acetate as eluent to give the desired product (1.12 g, 32% yield).
¹H-NMR: (300MHz, CDCl₃) δ 8.52 (d, 2H, J=3Hz), 7.39 (d, 2H, J=9Hz) 7.18 (d, 2H, J=6Hz) 6.79 (d, 2H, J=9Hz), 3.79-4.07 (m, 3H), 2.69-3.01 (m, 2H), 1.96 (m, 2H). LCMS: Mass Found [M+H]⁺ 322 Calcd for C₁₅H₁₇BrNO₂321.

### 3(iii) 1-(2'-Fluoro-5'-nitro-biophenyl-4-yloxy)-4-pyridin-4-yl-butan-2-ol (7)

A mixture of 6 (1 eqv) and 2-fluoro-5-nitrophenylboronic acid (1eqv) was taken in an oven-dried flask. Toluene and ethanol (4:1) were added and this solution was then added to aqueous sodium carbonate (1.9 vol of 2M) and then purged with nitrogen to remove the dissolved oxygen. The Palladium (0) catalyst (0.02eqv) was added to the reaction mixture, which was then heated to reflux for 1.5h. The reaction was partitioned between ethyl acetate and water. The organic phase was separated and washed with brine (2 x 25 mL) and dried over anhydrous sodium sulphate. The product was then purified by column chromatography on silica gel using hexane and ethyl acetate as eluent to give the desired purified product in a yield of 59%.
¹H-NMR: (400 MHz, CDCl₃) δ 8.53 (m, 2H), 8.37 (m, 0.5H), 8.20 (m, 0.5H), 7.54 (dd, 1 H,J=8Hz), 7.39 (dd, 1H, J=8Hz); 7.30 (m, 1 H), 7.20 (m, 1 H), 7.03 (dd, 1H, J=4Hz), 6.79 (dd, 1H, J=4Hz), 3.81-4.12 (m, 3H), 2.71-3.01 (m, 2H), 1.93 (m, 2H), LCMS: Mass Found [M+H]⁺ 382 Calcd for C₂₁H₁₉FN₂O₄ 381

### 3(iv) 3-[1-(2'-Fluoro-5'-nitro-biphenyl-4-yloxymethyl)-3-pyridin-4-yl-propyl]-thiazolidine-2,4-dione (Non-radioactive Imaging Agent 2)

To a solution of triphenylphosphine (2eqv) in anhydrous THF, was added diethyl azodicarboxylate (2eqv). The resulting orange solution was stirred for 10 minutes before the addition of 2,4-thiazolinedione (2eqv). The stirring was continued for another 15 minutes. To this reaction mass was added compound **7** (1eqv) and the reaction stirred for 2h. The reaction mixture was then concentrated under reduced pressure and product isolated by repeated column chromatography on silica gel using hexane and ethyl acetate as eluent to give the desired purified product in 96% yield.
¹H-NMR: (400 MHz, CDCl₃) δ 8.54 (d, 2H, J=4Hz), 8.36 (dd, 1H, J=8Hz, 4Hz), 8.21 (m, 1 H), 7.52 (d, 2H, J=8Hz), 7.29 (d, 2H, J=8Hz), 7.15 (d, 2H, J=4Hz), 6.98 (d, 2H, J=8Hz), 4.77 (m, 1H), 4.54 (t, 1H, J=12Hz), 4.21 (m, 1 H), 3.79 (s, 2H), 2.51-2.83 (m, 3H), 2.03-2.22 (m, 1H), LCMS: Mass Found [M+H]⁺ 482 Calcd for C₂₄H₂₀FN₃O₅S, 481.

### Example 2: Synthesis of Imaging Agent 2

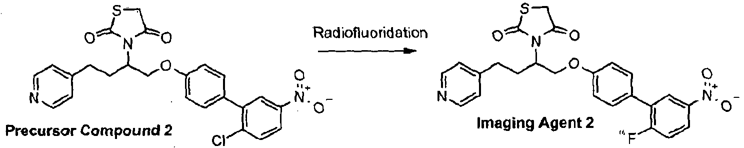

Precursor Compound 2 is prepared using the method as described above for Non-radioactive Imaging Agent 2, but where 1-(2'-Chloro-5'-nitro-biphenyl-4-yloxy)-4-pyridin-4-yl-butan-2-ol is synthesised in step (iii) instead of 1-(2'-Fluoro-5'-nitro-biphenyl-4-yloxy)-4-pyridin-4-yl-butan-2-ol. Radiofluoridation of Precursor Compound 2, e.g. using [F-18]fluoride in acetonitrile in the presence of potassium carbonate and Kryptofix, results in Imaging Agent 2.

### Reference Example 3: Synthesis of Non-radioactive Imaging Agent 3

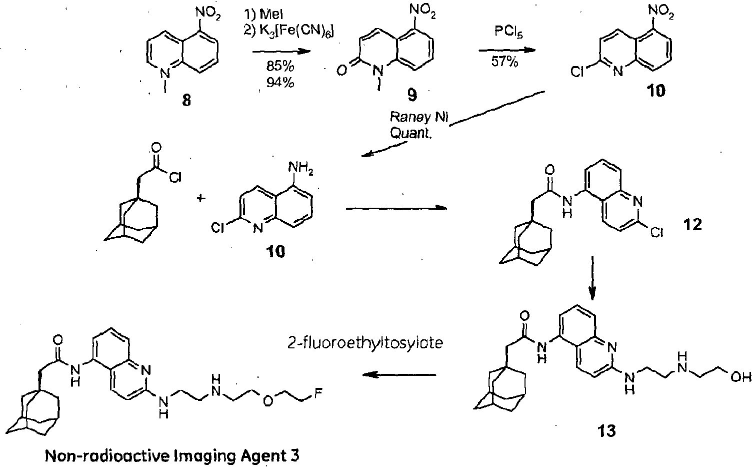

### 5(i) N-Methyl-5-nitroquinoline (8)

A mixture of 5-nitroquinoline (2.0 g, 11.49 mmol), dry toluene (2 mL) and dry methyl lodide (1 mL) was refluxed for 6h. The bright red crystalline methiodide was filtered and washed with toluene. The filtrate was concentrated and again refluxed with methyl lodide in toluene to obtain more material. The process was repeated until any further yield became negligible. Yield = 1500 mg (60%),
¹H-NMR (300 MHz, DMSO) δ = 9.70 (1 H, d, J = 6 Hz, Ar-H), 9.52 (1 H, d, J = 9 Hz, Ar-H), 8.93 (1 H, d, J = 9 Hz, Ar-H), 8.78 (1 H, d, J = 9 Hz, Ar-H), 8.46-8.36 (2H, m, Ar-H), 4.73 (3H, s, N-CH₃).
LCMS Mass Found = [M+H)⁺ 190, Calcd for C₁₀H₉N₂O₂ = 189.

### 5(ii) 1-Methyl-5-nitro-carbostyril (9)

N-Methylquinoline (5.0 g, 26.45 mmol) was dissolved in water (50 mL) and cooled to 0°C in an ice bath. Potassium ferricyanide (19.2 g, 58.19 mmol) in water (50 mL) and sodium hydroxide (5.3 g, 132.25 mmol) in water (8 mL) were added simultaneously with stirring. The base addition was completed in 10 minutes and oxidizing agent addition was completed in 30 minutes. The reaction mixture was stirred at 0°C for 90 minutes and at room temperature for 18h. A yellow crystalline precipitate of the carbostyril was filtered, washed with small amount of cold water and dried to give a yellow solid (3.55 g, 66% yield).
¹H-NMR (300 MHz, DMSO) δ = 8.15 (1H, d, J = 9 Hz, Ar-H), 7.94-7.78 (3H, m, Ar-H), 6.84 (1 H, d, J = 9Hz, Ar-H), 3.68 (3H, s, N-CH₃).
LCMS Mass Found = [M+H)⁺ 205, Calcd for C₁₀H₈N₂O₃ 204.

### 5(iii) 2-Chloro-5-nitroquinoline (10)

Phosphorous oxychloride (25 mL) was added slowly to a solution of carbostyril **9** (1.5 g, 7.35 mmol) in o-dichlorobenzene at 0°C and then refluxed for 12h. The reaction mixture was then quenched by pouring into the ice-cold water and then extracted with chloroform. Solvent was then dried over anhydrous sodium sulphate, filtered and evaporated to obtain a gummy residue. The product was obtained as a light brown solid by trituration with water (520 mg, 34% yield).
¹H-NMR (300 MHz, CDCl₃) δ = 9.02 (1 H, d, J = 9 Hz, Ar-H), 8.43 (1 H, d, J = 9 Hz, Ar-H), 8.36 (1H, d, J = 9 Hz, Ar-H), 7.87 (1H, t, J = 9 Hz, Ar-H, 7.66 (1 H, d, J = 9 Hz, Ar-H).
LCMS Mass Found = [M+H)⁺ 209, Calcd for C₉H₅N₂O₂Cl 208.

### 5(iv) 2-Chloro-5-aminoquinoline (11)

2-Chloro-5-nitroquinoline (300 mg, 1.44 mmol) was added to glacial acetic acid (3 mL) and stirred at 65°C. Iron powder (403 mg) was added to the mixture and stirring continued for 5 h. The reaction mixture was then cooled, concentrated and the residue diluted with water (20 mL) and pH was adjusted by addition of sodium carbonate solution. The product was then extracted with ethyl acetate (3 x 15 mL). The combined extractes were washed with brine, dried over anhydrous sodium sulphate, filtered and evaporated to give the product as a brown oil (260 mg >90% yield).
¹H-NMR (300 MHz, CDCl₃) δ = 8.11 (1 H, d, J = 9Hz, Ar-H), 7.55-7.44 (2H, m, Ar-H), 7.30 (1 H, d, J = 9Hz, Ar-H), 6.82 (1 H, d, J = 9 Hz, Ar-H).
LCMS Mass Found = [M+H)⁺ 179, Calcd for C₉H₇N₂Cl 178.

### 5(v) N-(2-Chloroquinolin-5-yl)-2-(adamantyl)acetamide (12)

To a solution of 1-adamantane acetic acid (423 mg, 2.18 mmol) in dry dichloromethane (15 mL), HOBt (98 mg, 0.725 mmol) and EDCI (350 mg, 1.81 mmol), were added and the reaction mixture was stirred for 1 h at 0°C. 2-Chloro-5-aminoquinoline (260 mg, 1.45 mmol) dissolved in dichloromethane (5 mL) was added followed by triethylamine (500 µl, 3.63 mmol) and the mixture was stirred overnight. The reaction mixture was then quenched with the addition of water and extracted with more dichloromethane. The combined organic layers were dried over anhydrous sodium sulphate, filtered and evaporated. The product was isolated by column chromatography on silica gel using ethyl acetate as eluent as a yellow-brown solid (460 mg 90% yield).
¹H-NMR (400 MHz, CD₃OD) δ = 8.41 (1 H, d, J = 8 Hz, Ar-H), 7.86-7.79 (2H, m, Ar-H), 7.71 (1 H, d, J = 8 Hz, Ar-H), 7.56 (1 H, d, J = 8 Hz, Ar-H), 2.29 (2H, s, CH₂), 2.03 (1H, bs, NH), 1.84-1.72 (15H, m, Adamantane H).
LCMS Mass Found = [M+H)⁺ 355, Calcd for C₂₁H₂₃ON₂Cl 354.

### 5(vi) N-(2-(2-(2-Hydroxyethylamino)quinolin-5-yl)-2-(adamantyl)acetamide (13)

N-(2-Chloroquinolin-5-yl)-2-(adamantyl)acetamide (**12**) (400 mg, 1.13 mmole) was dissolved in ethanol (8 mL) and N- (2-hydroxyethyl)-ethyelenediamine (4 mL) was added and the mixture refluxed for 14h. The solvent was removed and washed with cold water. The residue was then extracted with dichloromethane and washed again with saturated sodium carbonate solution. The organic layer was dried over anhydrous sodium sulfate and using petroleum ether added to precipitate the product as a brown crystalline solid (400 mg 83%yield).
LCMS Mass Found = [M+H)⁺ 423, Calcd for C₂₅H₃₄N₄O₂ 422

### 5(vii) 2-(1-Adamantyl)-N-(2-(2-fluoro ethoxy)ethyl)ethane-1,2diamino) amino) quinolin-5-yl)acetamide (Non-radioactive Imaging Agent 3)

N-(2-(2-(2-hydroxyethylamino)quinolin-5-yl)-2-(adamantyl)acetamide (**13**) (160 mg, 0.38 mmol) was dissolved in dry dimethylformamide (5 mL) and then cesium carbonate (135 mg, 0.42 mmol) was added. A dimethylformamide solution of fluoroethyltosylate (106 mg in 2 mL, 0.42 mmol) was added. The reaction mixture was then stirred at 55°C for 24 h, quenched with water and then extracted with ethyl acetate. The organic layer was then dried over anhydrous sodium sulphate, filtered and evaporated. The product was isolated by column chromatography on silica gel, using ethyl acetate as eluent, as a pale green solid (30 mg 20% yield).
¹H-NMR (400 MHz, CDCl₃) δ = 8.28 (1H, bs, Ar-H), 7.82 (1 H, bs, Ar-H), 7.35 (2H, bs, Ar-H), 6.58 (1 H, bs, Ar-H), 4.68 (1 H, bs, NH), 4.26 (2H, s, CH₂F), 3.80-3.43 (8H, m, CH₂), 2.22 (2H, s, CH₂CO), 2.00 (4H, s, CH₂), 1.83-1.60 (15H, m, Adamantane H).
¹H-NMR (400 MHz, CD₃OD) δ = 7.99 (1 H, d, J = 8 Hz, Ar-H), 7.52 (2H, m, Ar-H), 7.27 (1H, d, J = 8 Hz, Ar-H), 6.80 (1H, d, J = 8 Hz, Ar-H), 4.26 (2H, t, J = 8 Hz, CH₂F), 3.84-3.66 (6H, m, CH₂), 3.54 (2H, bs, CH₂), 2.25 (2H, s, CH₂CO), 2.02 (2H, s, CH₂), 1.81-1.73 (15H, m, Adamantane H).
LCMS Mass Found = [M+H)⁺ 469, Calcd for C₂₇H₃₇N₄O₂F 468.

### Reference Example 4: Synthesis of Imaging Agent 3

The method as described above for the synthesis of Non-radioactive imaging Agent 3 can be applied to obtain imaging Agent 3 by using 2-[¹⁸F]-fluoroethyltosylate in place of 2-fluoroethyltosylate in the final step.

### Reference Example 5: Synthesis of Non-radioactive Imaging Agent 4

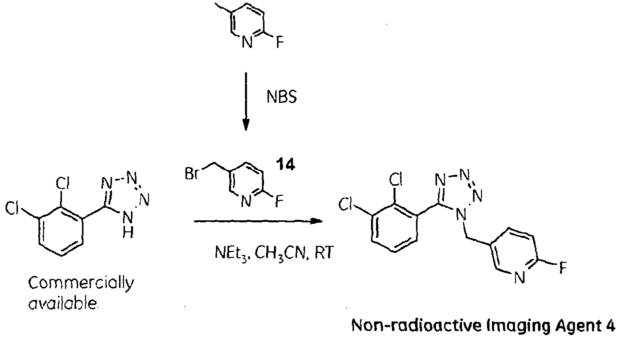

### 7(i) 3-bromomethyl-6-fluoropyridine (14)

2-Fluoro-5-methylpyridine (1.20 g, 10.8mmol) was added to a 100 mL oven dried round bottom flask, flushed with dry nitrogen. Dry carbon tetrachloride (50 mL) was added followed by NBS (1.92 g, 10.8mmol) and a catalytic amount *o*f AlBN. The reaction was heated at reflux for 5h. The succinimide was removed by filtration through celite and the carbon tetrachloride was removed by evaporation under reduced pressure. The product was isolated after column chromatography on silica gel [Eluent: 15-40% ethyl acetate-hexane] as a solid (1.15 g, 56% yield).
¹H-NMR: (300MHz, CD₃OD) δ 8.26 (s, 1H, Ar) 8.02 (t, 1H, J=9Hz, Ar) 7.02-7.12 (d, 1H, J=9Hz, Ar) 4.62 (s, 2H, CH2) LCMS: [M+H]⁺ 189/191 Calc. for C₆H₆BrFN 190.

### 7(ii) 5-[5-(2,3-Dichloro-phenyl)-tetrazol-1-ylmethyl]-2-fluoro-pyridine (Non-radioactive Imaging Agent 4)

To an oven dried, round bottom flask under dry nitrogen atmosphere were added 5-(2,3-dichloro-phenyl)-1H-tetrazole (0.8 g, 3.7mmol), dry dimethylformamide (8 mL). The solution was cooled to 0°C. Triethylamine (0.9 g, 8.9mmol) was added and stirred for 10 minutes followed by the addition of 3-bromomethyl-6-fluoropyridine (**14**) (0.85 g, 4.46mmol). The whole reaction mass was stirred at room temperature for 12h. Solvent was removed under reduced pressure and water (50 mL) added extracted with ethyl acetate (4 x 15 mL). The combined extracts were dried over anhydrous sodium sulphate, filtered and evaporated. The crude reaction mass was then purified by column chromatography on silica gel (eluent-30%-50% ethyl acetate- hexane gradient) to yield the desired 1,5-disubstituted compound (120 mg, 10% yield), which is in the minor amount and the 2,5-disubstituted compound as the major product.
¹H-NMR: (300MHz, CDCl₃) δ 7.91 (s, 1 H, Ar) 7.76 (dd, 1 H, Ar) 7.68 (m, 1 H, Ar) 7.39 (t, 1H, J=9Hz, Ar) 7.28 (s, 1H, Ar) 7.20 (dd, 1 H, Ar) 6.93 (dd, 1H, Ar) 5.48 (s, 2H, CH₂)
LCMS: Mass Found [M+H]⁺(324) Cacld for C₁₃H₈Cl₂FN₅ 323. Yield = 10%.

### Reference Example 6: Synthesis of Imaging Agent 4

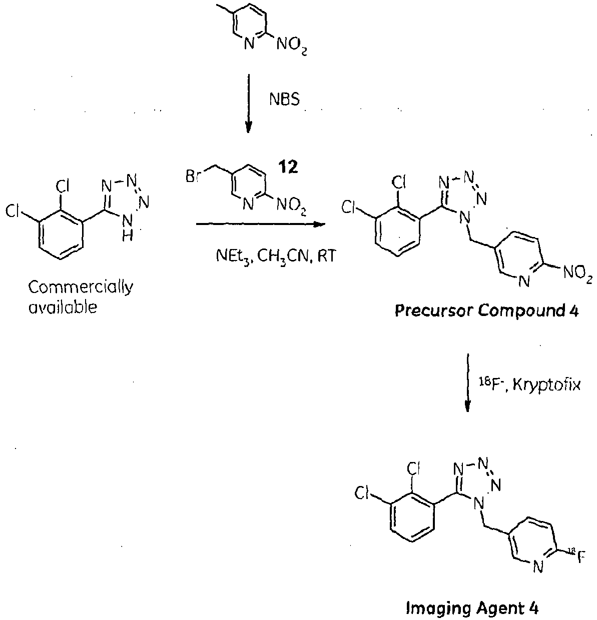

### 8(i) 5-[5-(2,3-Dichloro-phenyl)-tetrazol-1-ylmethyl]-2-nitro-pyridine (Precursor Compound 4)

Precursor Compound 4 was prepared in a yield of 12% using the method described in Example 7 for Non-radioactive Imaging Agent 4, but where 3-bromomethyl-6-nitroropyridine was used instead of 3-bromomethyl-6-fluoropyridine.
¹H-NMR: (300MHz, CDCl₃) δ 8.38 (s, 1 H, Ar) 8.27 (d, 1 H, J=9Hz, Ar) 7.95 (d, 1H, J=9Hz, Ar); 7.78 (d, 1H, J=6Hz, Ar) 7.42 (t, 1H, J=9Hz, Ar) 7.28 (s, 1 H, Ar) 5.62 (s, 2H, CH2).
LCMS: Mass Found [M+H]⁺ 351 Cacld for C₁₃H₈Cl₂N₆O₂ 350.

### 8(ii) 5-[5-(2,3-Dichloro-phenyl)-tetrazol-1-ylmethyl]-2-[¹⁸F]-fluoro-pyridine

### (Imaging Agent 4)

Precursor Compound 4 can be radiofluoridated using e.g. [F-18]fluoride in acetonitrile in the presence of potassium carbonate and Kryptofix to provide Imaging Agent 4.

### Reference Example 7: Synthesis of Non-radioactive Imaging Agent 5

Non-radioactive Imaging Agent 5 was prepared as described in Example 7 for Non-radioactive Imaging Agent 4, but where but where 3-bromomethyl-2-fluororopyridine was used instead of 3-bromomethyl-6-fluoropyridine.
¹H-NMR: (300MHz, CDCl₃) δ 8.22 (d, 1H, J=3Hz, Ar) 7.73 (m, 2H, Ar) 7.4 (t, 1H, J=9Hz, Ar) 7.3 (m, 2H, Ar) 7.22 (m, 1H, Ar) 5.5 (s, 2H, CH₂).
LCMS: Mass Found [M+H]⁺ 324 Cacld for C₁₃H₈Cl₂FN₅323

### Reference Example 8: Synthesis of Imaging Agent 5

### 10(i) 5-[5-(2,3-Dichloro-phenyl)-tetrazol-1-ylmethyl]-2-nitro-pyridine (Precursor Compound 5)

Precursor Compound 5 was prepared in 11 % yield using the method described in Example 7 for Non-radioactive Imaging Agent 4, but where 3-bromomethyl-2-nitroropyridine was used instead of 3-bromomethyl-6-fluoropyridine.
¹H-NMR: (300MHz, CDCl₃) 5 8.65 (d, 1H, J=3Hz, Ar) 7.68-7.86 (m, 3H, Ar) 7.35-7.50(m, 2H, Ar) 5.79 (s, 2H, CH2).
LCMS: Mass Found [M+H]⁺ 351 Calcd for C₁₃H₈Cl₂N₆O₂. 350.

### 10(ii) 5-[5-(2,3-Dichloro-phenyl)-tetrazol-1-ylmethyl]-6-[¹⁸F]-fluoro-pyridine (Imaging Agent 5)

Precursor Compound 5 can be radiofluoridated using e.g. [F-18]fluoride in acetonitrile in the presence of potassium carbonate and Kryptofix to provide Imaging Agent 5.

### Example 9: Pore-forming Assay to determine P2X₇ Binding

The assay method used was based on the ability of the DNA binding dye, Yo Pro-1 (quinolinium, 4[3-methyl-2(3H)-benzoxazolylidene) methyl]-1-[3-(trimethyl-ammonio) propyl]-dioxide) to enter through the dilated or "large pore form" of the P2X₇ receptor and to bind to intracellular DNA/RNA whereupon it increases fluorescence intensity. Yo Pro-1 was therefore used to quantify inhibition of P2X₇ function. This assay was based on the methods published by Michel et al., (B.J.Pharmacol 1998; 125: 1194-1201).

Initially, HEK.293 cells were transiently transfected using Lipofectamine TML TX (Invitrogen) for 72hrs with P2X₇ cDNA. 48 hours prior to use the cells were seeded into poly-D-lysine coated 96-well black-walled, clear bottomed plates, at a density of 30,000 cells/well. Stock solutions of each test compound were prepared at a concentration of 40mM in 100% DMSO

Following the 48 hour incubation the culture medium was removed from the transfected cells, the cells were washed once and placed in pre-warmed sucrose assay buffer (Sucrose: 280mM, KCI: 5mM, CaCl2: 0.5mM, glucose: 10mM, HEPES: 10mM, N-methyl-D-glucamine: 10mM; pH7.4). The test compounds were added to the plate at a concentration of 10µM and 100nM in triplicate and incubated at 37°C for 30 minutes. The final DMSO concentration in the assay was 1%. After this time Yo Pro-1 dye and Bz-ATP solution was added at concentrations of 1µM and 30µM respectively for 60 minutes at 37°C. The fluorescence was then read at 485 nM excitation and 530 nM emission.

The non-selective P2X channel antagonist PPADS was used as a reference inhibitor in the assay. A dose-response to PPADS was performed on the assay plate using a starting concentration of 200µM followed by a 1 in 6 serial dilution covering 200µM to 0.4nM. For each compound data set, a percentage inhibition value was calculated based on the three assay points generated.

## Claims

1. An *in vivo* imaging agent suitable for *in vivo* imaging of the central nervous system (CNS) of a subject, wherein said *in vivo* imaging agent is a compound of Formula II, or a salt or solvate thereof, wherein:
Formula II is defined as follows: 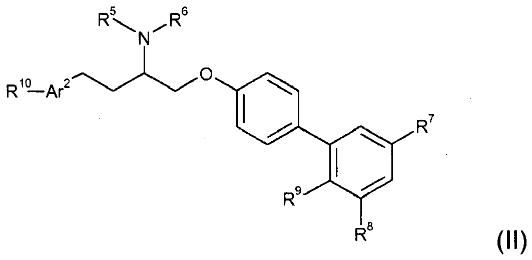
wherein one of R⁵-R¹⁰ comprises an *in vivo* imaging moiety which is a gamma-emitting radioactive halogen or a positron-emitting radioactive non-metal, and wherein:
R⁵ and R⁶ are independently selected from hydrogen, C₁₋₆ alkyl, C₁₋₆ fluoroalkyl, C₁₋₆ acyl, C₁₋₆ fluoroacyl, C₁₋₆ carboxylic acid alkyl ester, C₁₋₆ alkoxy, C₁₋₆ fluoroalkoxy; or R¹ and R² taken together with the nitrogen to which they are attached, form a 5- or 6-membered nitrogen-containing heterocycle optionally comprising another heteroatom selected from nitrogen, sulfur or oxygen, and optionally having 1 or 2 oxo groups on the ring;
R⁷-R⁹ are independently selected from hydrogen, halo, nitro, C₁₋₆ alkyl, C₁₋₆ haloalkyl, C₅₋₆ aryl, or C₅₋₆ haloaryl;
R¹⁰ is selected from hydrogen, hydroxyl, nitro, halo, or is the group C(=O)NR¹¹R¹² wherein R¹¹ and R¹² are as defined for R⁵ and R⁶; and,
Ar² is a 5- to 6-membered aryl group having 0-3 heteroatoms selected from nitrogen, oxygen and sulfur;

2. The *in vivo* imaging agent of Formula II as defined in Claim 1, which is a compound of Formula II*: 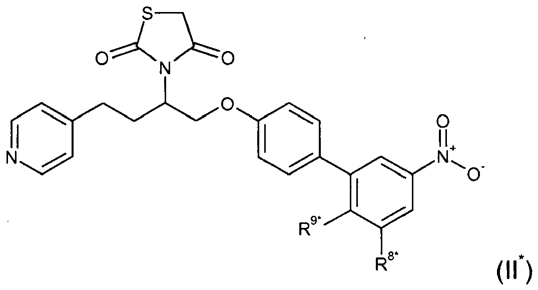 wherein one of R^{8*} and R^{9*} is ¹⁸F and the other is hydrogen.

3. The *in vivo* imaging agent as defined in Claim 1 or Claim 2 wherein said *in vivo* imaging moiety is selected from ¹²³I, ¹¹C and ¹⁸F.

4. A method for the synthesis of an *in vivo* imaging agent of Formula II as defined in Claim 1, wherein said method comprises reaction of a suitable source of said *in vivo* imaging moiety with a precursor compound of Formula IIa: 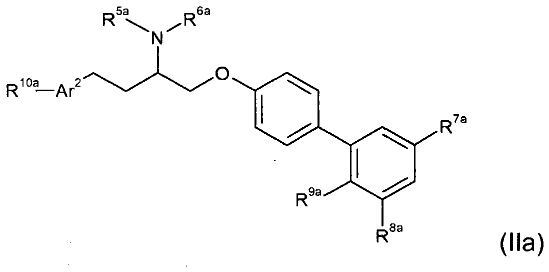 wherein:
R^{5a} and R^{6a} are as defined in Claim 1 for R⁵ and R⁶, respectively; and,
one of R^{7a}-R^{10a} represents a precursor group, and the remainder are as defined in Claim 1 for R⁷-R¹⁰.

5. The method as defined in Claim 4 wherein said precursor compound of Formula IIa is a compound of Formula IIa*: 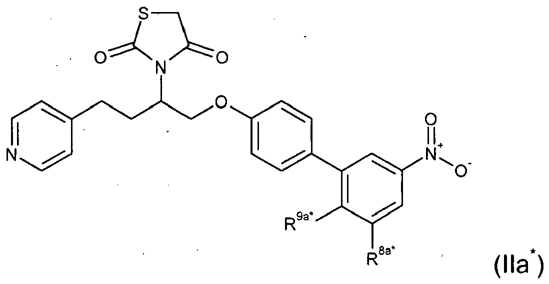 wherein one of R^{8a*} and R^{9a*} is a precursor group, and the other is hydrogen.

6. The method as defined in Claim 4 or Claim 5 wherein said method is automated.

7. A cassette for carrying out the method as defined in Claim 6 comprising:
(i) a vessel containing a precursor compound as defined in the method of Claim 4 or Claim 5; and
(ii) means for eluting the vessel with a suitable source of an *in vivo* imaging moiety, said *in vivo* imaging moiety as defined in either Claim 1 or Claim 3.

8. The cassette as defined in Claim 7 which additionally comprises:
(iii)an ion-exchange cartridge for removal of excess *in vivo* imaging moiety; and optionally,
(iv)a cartridge for deprotection of the resultant radiolabelled product to form an *in vivo* imaging agent as defined herein.

9. A method of imaging a subject to facilitate the determination of the presence, location and/or amount of P2X₇ receptors in the CNS of a subject, said method comprising the following steps:
(i) providing a subject to whom a detectable quantity of an *in vivo* imaging agent as defined in any one of Claims 1-3 has been administered;
(ii) allowing the *in vivo* imaging agent to bind to P2X₇ receptors in said subject;
(iii) detection of signals emitted by said *in vivo* imaging agent by an *in vivo* imaging method; and,
(iv) generation of an image representative of the location and/or amount of said signals.

10. The method as defined in Claim 9 wherein said subject is an intact mammalian body *in vivo.*

11. The *in vivo* imaging agent as defined in any one of Claims 1-3 for use in a method as defined in either of Claims 9 or 10 wherein said subject is known or suspected to have a pathological condition associated with abnormal expression of P2X₇ receptors in the CNS.

12. The *in vivo* imaging agent as defined in any one of Claims 1-3 for use in a method of diagnosis comprising steps (i)-(iv) of Claim 9, and further comprising the following step:
(v) evaluating the image generated in step (iv) to diagnose a pathological condition associated with abnormal expression of P2X₇ receptors in the CNS (a "P2X₇ condition").

13. The *in vivo* imaging agent as defined in any one of Claims 1-3 for use in a method for the determination of the presence, location and/or amount of inflammation in the CNS of a subject.

14. The *in vivo* imaging agent as defined in any one of Claims 1-3 for use in the preparation of a medicament for the determination of the presence, location and/or amount of inflammation in the CNS of a subject.

15. A radiopharmaceutical composition which comprises the *in vivo* imaging agent as defined in any one of Claims 1-3 together with a biocompatible carrier, in a form suitable for mammalian administration.

## Patentansprüche

1. *In*-*vivo*-Bildgebungsmittel, geeignet zur *In-vivo*-Bildgebung des Zentralnervensystems (ZNS) eines Lebewesens, wobei das *In-vivo-*Bildgebungsmittel eine Verbindung der Formel II ist, oder ein Salz oder Solvat derselben, wobei:
Formel II wie folgt definiert ist: 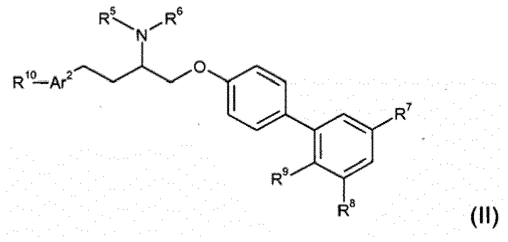
worin eines von R⁵-R¹⁰ eine In-vivo-Bildgebungseinheit umfasst, die ein gammastrahlendes radioaktives Halogen oder ein positronenstrahlendes radioaktives Nichtmetall ist, und worin:
R⁵ und R⁶ unabhängig voneinander gewählt sind aus Wasserstoff, C₁₋₆-Alkyl, C₁₋₆-Fluoralkyl, C₁₋₆-Acyl, C₁₋₆-Fluoracyl, C₁₋₆-Carbonsäurealkylester, C₁₋₆-Alkoxy, C₁₋₆-Fluoralkoxy; oder R¹ und R², zusammengenommen mit dem Stickstoff, an den sie gebunden sind, einen 5- oder 6-gliedrigen stickstoffhaltigen Heterozyklus bilden, der gegebenenfalls ein weiteres Heteroatom, gewählt aus Stickstoff, Schwefel oder Sauerstoff, umfasst und gegebenenfalls 1 oder 2 Oxogruppen auf dem Ring aufweist;
R⁷-R⁹ unabhängig voneinander gewählt sind aus Wasserstoff, Halogen, Nitro, C₁₋₆-Alkyl, C₁₋₆-Haloalkyl, C₅₋₆-Aryl oder C₅₋₆-Haloaryl;
R¹⁰ gewählt ist aus Wasserstoff, Hydroxyl, Nitro, Halogen, oder steht für die Gruppe C(=O)NR¹¹R¹², worin R¹¹ und R¹² die für R⁵ und R⁶ angegebene Bedeutung haben; und
Ar² für eine 5- bis 6-gliedrige Arylgruppe mit 0-3 Heteroatomen steht, die aus Stickstoff, Sauerstoff und Schwefel gewählt sind.

2. In-vivo-Bildgebungsmittel der Formel II nach Anspruch 1, das eine Verbindung der Formel II* ist: 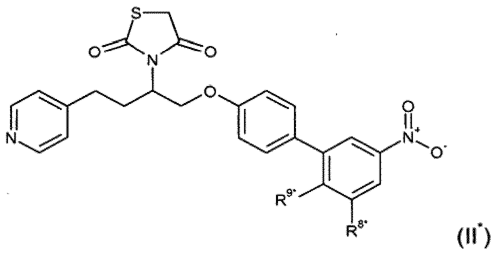 worin das eine von R^{8*} und R^{9*} für ¹⁸F steht und das andere für Wasserstoff steht.

3. *In-vivo*-Bildgebungsmittel nach Anspruch 1 oder Anspruch 2, wobei die *In-vivo-*Bildgebungseinheit aus ¹²³I, ¹¹C und ¹⁸F gewählt ist.

4. Verfahren zur Synthese eines *In-vivo*-Bildgebungsmittels der Formel II nach Anspruch 1, wobei das Verfahren die Umsetzung einer geeigneten Quelle der *In-vivo*-Bildgebungseinheit mit einer Präkursorverbindung der Formel IIa umfasst: 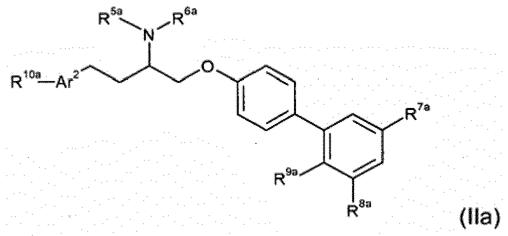 worin:
R^{5a} und R^{6a} die in Anspruch 1 für jeweils R⁵ und R⁶ angegebene Bedeutung haben; und
eines von R^{7a}-R^{10a} eine Präkursorgruppe darstellt, und der Rest die in Anspruch 1 für R⁷-R¹⁰ angegebene Bedeutung hat.

5. Verfahren nach Anspruch 4, wobei die Präkursorverbindung der Formell IIa eine Verbindung der Formel IIa* ist: 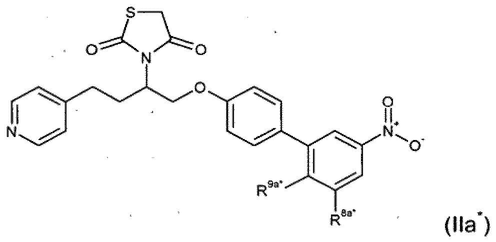 worin das eine von R^{8a*} und R^{9a*} für eine Präkursorgruppe steht, und das andere für Wasserstoff steht.

6. Verfahren nach Anspruch 4 oder Anspruch 5, wobei das Verfahren automatisiert ist.

7. Kassette zum Ausführen des Verfahrens nach Anspruch 6, umfassend:
(i) ein Gefäß, das eine Präkursorverbindung, wie im Verfahren aus Anspruch 4 oder Anspruch 5 definiert, enthält; und
(ii) Mittel zum Eluieren des Gefäßes mit einer geeigneten Quelle einer *In-vivo*-Bildgebungseinheit, wobei die *In-vivo*-Bildgebungseinheit entweder wie in Anspruch 1 oder wie in Anspruch 3 definiert ist.

8. Kassette nach Anspruch 7, die zusätzlich umfasst:
(iii) eine Ionenaustauschkartusche zur Entfernung überschüssiger *In-vivo-*Bildgebungseinheit; und gegebenenfalls
(iv) eine Kartusche zur Entschützung des resultierenden radioaktiv markierten Produkts zwecks Bildung eines *In-vivo*-Bildgebungsmittels, wie hierin definiert.

9. Verfahren zur Bildgebung eines Lebewesens, um die Bestimmung des Vorhandenseins, des Ortes und/oder der Menge von P2X₇-Rezeptoren im ZNS eines Lebewesens zu erleichtern, wobei das Verfahren die folgenden Schritte umfasst:
(i) Zur-Verfügung-Stellen eines Lebewesens, dem eine erfassbare Menge eines *In-vivo*-Bildgebungsmittels nach einem der Ansprüche 1-3 verabreicht worden ist;
(ii) Bindenlassen des *In-vivo*-Bildgebungsmittels an P2X₇-Rezeptoren in dem Lebewesen;
(iii) Erfassen von Signalen, die von dem *In-vivo*-Bildgebungsmittel ausgesendet werden, durch ein *In-vivo*-Bildgebungsverfahren; und
(iv) Erzeugen eines Bildes, das den Ort und/oder die Menge der Signale repräsentiert.

10. Verfahren nach Anspruch 9, wobei das Lebewesen ein intakter Säugerkörper *in vivo* ist.

11. *In*-*vivo*-Bildgebungsmittel nach einem der Ansprüche 1-3 zur Verwendung in einem Verfahren nach einem der Ansprüche 9 oder 10, wobei von dem Lebewesen bekannt ist oder vermutet wird, dass es einen pathologischen Zustand aufweist, der mit abnormer Expression von P2X₇-Repzeptoren im ZNS assoziiert ist.

12. *In-vivo*-Bildgebungsmittel nach einem der Ansprüche 1-3 zur Verwendung in einem Diagnoseverfahren, das die Schritte (i)-(iv) aus Anspruch 9 umfasst, und das weiterhin den folgenden Schritt umfasst:
(v) Evaluieren des in Schritt (iv) erzeugten Bildes zwecks Diagnose eines mit abnormer Expression von P2X₇-Rezeptoren im ZNS assoziierten pathologischen Zustands (eines "P2X₇-Zustands").

13. *In-vivo*-Bildgebungsmittel nach einem der Ansprüche 1-3 zur Verwendung in einem Verfahren für die Bestimmung des Vorhandenseins, des Ortes und/oder des Ausmaßes einer Entzündung im ZNS eines Lebewesens.

14. *In-vivo*-Bildgebungsmittel nach einem der Ansprüche 1-3 zur Verwendung in der Herstellung eines Medikaments für die Bestimmung des Vorhandenseins, des Ortes und/oder des Ausmaßes einer Entzündung im ZNS eines Lebewesens.

15. Radiopharmazeutische Zusammensetzung, die das *In-vivo*-Bildgebungsmittel nach einem der Ansprüche 1-3, zusammen mit einem biokompatiblen Träger, in einer zur Verabreichung an Säuger geeigneten Form umfasst.

## Revendications

1. Agent d'imagerie *in vivo* approprié pour l'imagerie *in vivo* du système nerveux central (SNC) d'un sujet, dans lequel ledit agent d'imagerie *in vivo* est un composé de formule II, ou un sel ou solvate de celui-ci, dans lequel :
la formule II est définie comme suit : 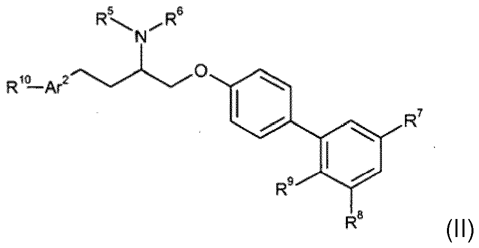
dans laquelle l'un de R⁵ à R¹⁰ comprend une fraction d'imagerie *in vivo* qui est un halogène radioactif émetteur de rayon gamma ou un élément non métallique radioactif émetteur de positron, et dans laquelle :
R⁵ et R⁶ sont sélectionnés indépendamment à partir de l'hydrogène, un alkyle en C₁₋₆, un fluoro-alkyle en C₁₋₆, un acyle en C₁₋₆; un fluoro-acyle en C₁₋₆, un ester d'alkyle d'acide carboxylique en C₁₋₆, un alkoxy en C₁₋₆, un fluoro-alkoxy en C₁₋₆ ; ou R¹ et R², considérés ensemble avec l'azote auquel ils sont liés, forment un hétérocycle contenant de l'azote à 5 ou 6 éléments comprenant, en variante, un autre hétéroatome sélectionné à partir de l'azote, du soufre ou de l'oxygène, et comportant, en variante, 1 ou 2 groupes oxo sur le cycle ;
R⁷ à R⁹ sont sélectionnés indépendamment à partir de l'hydrogène, un groupe halo, nitro, un alkyle en C₁₋₆, un halo-alkyle en C₁₋₆, un aryle en C₅₋₆, ou un halo-aryle en C₅₋₆ ;
R¹⁰ est sélectionné à partir de l'hydrogène, un groupe hydroxyle, nitro, halo, ou
représente le groupe C(=O)NR¹¹R¹² dans lequel R¹¹ et R¹² sont tels que définis pour R⁵ et R⁶ ; et
Ar² est un groupe aryle à 5 ou 6 éléments comportant de 0 à 3 hétéro-atomes sélectionnés à partir de l'azote, de l'oxygène et du soufre.

2. Agent d'imagerie *in vivo* de formule II selon la revendication 1, qui est un composé de formule II* : 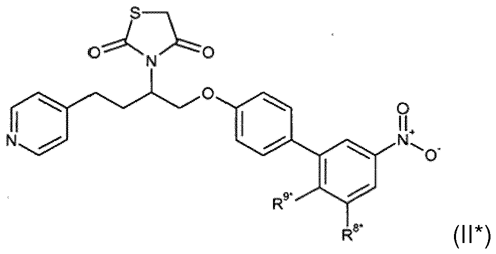 dans laquelle l'un de R^{8*} et R^{9*} représente le ¹⁸F et l'autre représente l'hydrogène.

3. Agent d'imagerie *in vivo* selon la revendication 1 ou 2, dans lequel ladite fraction d'imagerie *in vivo* est sélectionnée à partir de ¹²³I, ¹¹C et ¹⁸F.

4. Procédé de synthèse d'un agent d'imagerie *in vivo* de formule II selon la revendication 1, dans lequel ledit procédé comprend la réaction d'une source appropriée de ladite fraction d'imagerie *in vivo* avec un composé précurseur de formule IIa : 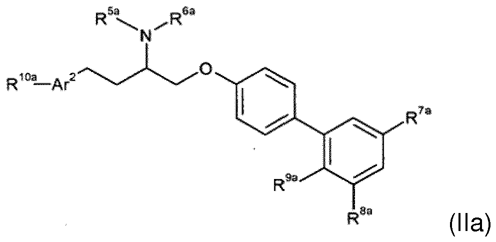 dans laquelle :
R^{5a} et R^{6a} sont tels que définis dans la revendication 1 respectivement pour R⁵ et R⁶ ; et
l'un de R^{7a} à R^{10a} représente un groupe précurseur, et le autres sont tels que défini dans la revendication 1 pour R⁷ à R¹⁰.

5. Procédé selon la revendication 4, dans lequel ledit composé précurseur de formule IIa est un composé de formule IIa* : 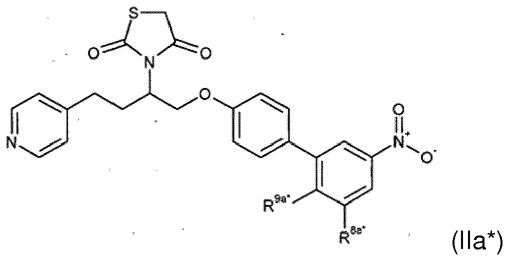 dans laquelle l'un de R^{8a*} et R^{9a*} représente un groupe précurseur, et l'autre représente l'hydrogène.

6. Procédé selon la revendication 4 ou 5, dans lequel ledit procédé est automatisé.

7. Cassette destinée à mettre en oeuvre le procédé selon la revendication 6 comprenant :
(i) une cuve contenant un composé précurseur tel que défini dans le procédé selon la revendication 4 ou 5 ; et
(ii) un moyen destiné à éluer la cuve avec une source appropriée d'une fraction d'imagerie *in vivo,* ladite fraction d'imagerie *in vivo* étant telle que définie selon la revendication 1 ou 3.

8. Cassette selon la revendication 7, qui comprend, en outre :
(ii) une cartouche d'échange d'ion destinée à extraire l'excès de fraction d'imagerie *in vivo* ; et en variante,
(iv) une cartouche destinée à assurer la déprotection du produit radiomarqué résultant afin de former un agent d'imagerie *in vivo* tel que défini ici.

9. Procédé d'imagerie d'un sujet destiné à faciliter la détermination de la présence, l'emplacement et/ou de la quantité de récepteurs P2X₇ du système SNC d'un sujet, ledit procédé comprenant les étapes suivantes :
(i) de préparation d'un sujet auquel une quantité détectable d'un agent d'imagerie *in vivo* selon l'une quelconque des revendications 1 à 3 a été administrée ;
(ii) d'attente de la liaison de l'agent d'imagerie *in vivo* aux récepteurs P2X₇ sur ledit sujet ;
(iii) de détection des signaux émis par ledit agent d'imagerie *in vivo* par un procédé d'imagerie *in vivo* ; et,
(iv) de génération d'une image représentative de l'emplacement et/ou du niveau desdits signaux.

10. Procédé selon la revendication 9, dans lequel ledit sujet est un corps mammifère entier *in vivo.*

11. Agent d'imagerie *in vivo* selon l'une quelconque des revendications 1 à 3, destiné à être utilisé dans un procédé selon l'une ou l'autre des revendications 9 ou 10, dans lequel il est connu ou suspecté que ledit sujet présente un état pathologique associé à une expression anormale de récepteurs P2X₇ dans le système SNC.

12. Agent d'imagerie *in vivo* selon l'une quelconque des revendications 1 à 3, destiné à être utilisé dans un procédé de diagnostic comprenant les étapes (i) à (iv) selon la revendication 9, et comprenant, en outre, l'étape suivante :
(v) d'évaluation de l'image générée à l'étape (iv) afin de diagnostiquer un état pathologique associé à l'expression anormale de récepteurs P2X₇ dans le système SNC (un "état P2X₇").

13. Agent d'imagerie *in vivo* selon l'une quelconque des revendications 1 à 3, destiné à être utilisé dans un procédé de détermination de la présence, de l'emplacement et/ou du niveau d'une inflammation dans le système SNC d'un sujet.

14. Agent d'imagerie *in vivo* selon l'une quelconque des revendications 1 à 3, destiné à être utilisé lors de la préparation d'un médicament destiné à déterminer la présence, l'emplacement et/ou le niveau d'une inflammation dans le système SNC d'un sujet.

15. Composition pharmaceutique qui comprend l'agent d'imagerie *in vivo* selon l'une quelconque des revendications 1 à 3, ensemble avec un support biocompatible, sous une forme appropriée à l'administration mammifère.
